# EUROPEAN PATENT SPECIFICATION

(11) **EP 2 316 420 B1**
(45) Date of publication and mention of the grant of the patent: **26.02.2014**
(21) Application number: 09173740.3
(22) Date of filing: 22.10.2009
(51) Int. Cl.: A61K 9/00, A61K 31/165, A61K 31/167, A61K 31/195, A61K 31/55, A61P 29/00

(54) **Topical ophthalmic composition to reduce pain**
Topische Ophthalmische Zusammensetzung zur Schmerzlinderung
Composition ophthalmique topique pour réduire la douleur

(43) Date of publication of application: 04.05.2011
(73) Proprietor: Consorzio Universitario Unifarm, 95125 Catania (IT)
(72) Inventor: Drago, Filippo, 95125 CATANIA (IT); Bucolo, Claudio, 95125 CATANIA (IT)
(74) Representative: Minoja, Fabrizio

(56) References cited:
- EP-A1- 1 700 616
- WO-A1-01/68082
- WO-A2-2009/145842
- US-B1- 6 350 781
- NISSMAN ET AL: "Oral Gabapentin for the Treatment of Postoperative Pain after Photorefractive Keratectomy" AMERICAN JOURNAL OF OPHTHALMOLOGY, OPHTHALMIC PUBL, CHICAGO, IL, US, vol. 145, no. 4, 28 January 2008 (2008-01-28), pages 623-629.E1, XP022561326 ISSN: 0002-9394
- C-S. KAVALIERATOS, T. DIMOU: "Gabapentin therapy for painful, blind glaucomatous eye: case report" PAIN MEDICINE, vol. 9, no. 3, 2008, pages 377-378, XP002564703

## Description

The invention concerns pharmaceutical compositions for the topical ophthalmic administration comprising an anticonvulsivant compound as the active ingredient and to the use thereof for the treatment of ocular pain and/or ocular inflammation.

### BACKGROUND OF THE INVENTION

Pain is a perceived nociceptive response to local stimuli in the body. The perception of pain at the level of the central nervous system requires the transmission of painful stimuli by peripheral sensory nerve fibers. Upon stimulation of tissue (i.e., thermal, mechanical or chemical), electro-chemical signals are transmitted from the sensory nerve endings to the spinal column, and hence to the brain where pain is perceived. The cornea is highly innervated with sensory afferents which transmit various painful stimuli to the central nervous system. Pain conditions involving the eye, therefore, can arise in numerous instances, such as: foreign body stimulus, inflammation, herpes, dry eye syndrome, accidental trauma, surgical procedures and post- surgical recovery. For example, ocular pain can result from photorefractive keratotomy ("PRK"), a vision correcting, surgical procedure whereby a laser is used to shape the cornea. This process involves the photoablation of Bowman's membrane and the stromal levels of the cornea. As a result, the denuding of the nerve-containing epithelial layers of the cornea can cause some patients to experience pain following laser surgery until the epithelium regenerates. Another example is the post-herpetic neuralgia, emergency department care of herpes zoster ophthalmicus (HZO) includes local wound care, adequate analgesia, starting antiviral agents, and antibiotics for secondary bacterial infection; further, when the blinking reflex and eyelid function are compromised, an eye lubricant is needed to prevent corneal desiccation injury.

Various therapies have been attempted for the alleviation of ocular pain. The use of non-steroidal anti-inflammatory drugs (NSAIDs), such as diclofenac, has been proposed to treat pain. These agents inhibit cyclooxygenase dependent prostaglandin synthesis. Prostaglandins can modulate pain perception at the level of the central nervous system and systemic administration of NSAIDs is known to provide analgesia. However, the use of NSAIDs can involve undesired side effects including gastrointestinal bleeding and kidney dysfunction. Local anesthetics are another class of pain modulators that relieve ocular pain by directly inhibiting nerve cellular function. One problem with local anesthetic therapy is that the anesthetics exhibit a short duration of action. Another problem with the use of local anesthetics is that their mechanism of action, non-specific membrane stabilization, can have the undesired coincident effect of also inhibiting biological functions of other cells, such as fibroblasts and surrounding neural cells. Therefore, even though pain sensation can be abated with local anesthetic treatment, healing and normal function of the tissue may be significantly compromised. There is a need, therefore, to discover agents which potently and specifically inhibit the transmission of painful stimuli by sensory afferents, without local anesthetic activity, following topical ocular application.

In addition to treating ocular pain, local topical ocular application of anesthetics has been proposed to reduce or eliminate sensations on the ocular surface to treat the symptoms of dry eye. However, chronic use of local anesthetics is accompanied by toxic side effects.

Gabapentin is known to treat ocular pain after photorefractive keratectony (Nissaman et al, Am. J. Ophthalmology 2008, 145, 623-629) and to treat painful, blind glaucomatous eye (Kavalieratas et al, Pain Medicine 2008, 9(3), 377-378). Capsaicin is also known in the treatment of ocular pain (WO 01/68082).

### SUMMARY OF THE INVENTION

It has now been found that gabapentin, administered topically, is particularly effective in the treatment of ocular pain in combination with local anaesthetic and capsaicin. The present invention accordingly concerns pharmaceutical compositions for the topical ophthalmic administration comprising said combination as the active ingredient and at least one pharmaceutically acceptable carrier, adjuvant and/or diluent.

The invention also concerns the use of the combination for the preparation of a topical ophthalmic medicament for the treatment of ocular pain and/or ocular inflammation.

### DETAILED DESCRIPTION OF THE INVENTION

The ophthalmic compositions of the invention comprise gabapentin in combination with a local anaesthetic such as lidocaine and capsaicin.

The compositions of the invention, in addition to an effective amount of the anticonvulsivant may also contain hyaluronic acid or a salt thereof. Hyaluronic acid is useful to prevent corneal desiccation injury.

The pain relief effect of gabapentin is enhanced by the combination of lidocaine with capsaicin to produce a temporal functional denervation preventing pain and neurogenic inflammation to the eye and ocular adnexa while the acute pain-producing effects of capsaicin are blocked by pretreatment with a local anaesthetic.

According to the present invention, said composition for topical ophthalmic administration or implantation into the conjunctival sac or anterior chamber of the eye is administered to a mammal in need thereof. The compositions are formulated in accordance with known methods for the desired route of administration. The compositions comprise a pharmaceutically effective amount of gabapentin.

A "pharmaceutically effective amount" refers to that amount of gabapentin that prevents or alleviates ocular pain and/or is sufficient to reduce or eliminate symptoms of dry eye. Preferably, compositions are intended to be administered topically to the eye in the form of eye drops or eye ointments, wherein the total amount of gabapentin will be about 0.001 to 5.0% (w/v). Preferably, the amount of gabapentin is about 0.01 to about 5.0% (w/v). Preferably, the compositions of the invention will be formulated as solutions, suspensions, creams, lotions, liposomes, eye drops or eye drop devices and other dosage forms for topical administration. Aqueous solutions are generally preferred, based on ease of formulation, as well as a patient's ability to easily administer such compositions by means of instilling one to two drops of the solutions in the affected eyes. However, the compositions may also be suspensions, viscous or semi-viscous gels, or other types of solid or semi-solid compositions. Suspensions may be preferred for cytokine synthesis inhibitors which are sparingly soluble in water. The compositions may also include various other ingredients, such as surfactants, tonicity agents, buffers, preservatives, co-solvents and viscosity building agents. Various tonicity agents may be employed to adjust the tonicity of the composition, preferably to that of natural tears for ophthalmic compositions. For example, sodium chloride, potassium chloride, magnesium chloride, calcium chloride, dextrose and/or mannitol may be added to the composition to approximate physiological tonicity. Such an amount of tonicity agent will vary, depending on the particular agent to be added. In general, however, the compositions will have a tonicity agent in an amount sufficient to cause the final composition to have an ophthalmically acceptable osmolality (generally about 150-450 mOsm, preferably 250-350 mOsm). An appropriate buffer system (e.g., sodium phosphate, sodium acetate, sodium citrate, sodium borate or boric acid) may be added to the compositions to prevent pH shift under storage conditions. The particular concentration will vary, depending on the selected agent. Preferably, however, the buffer will be chosen to maintain a target pH within the range of pH 6.0 - 7.5. Topical ophthalmic products may also be packaged in multidose form. Preservatives may thus be required to prevent microbial contamination during use. Suitable preservatives include: chlorobutanol, methyl paraben, propyl paraben, phenylethyl alcohol, edetate disodium, sorbic acid, polyquaternium-1, or other agents known to those skilled in the art. Such preservatives are typically used at a level of from 0.001 to 5.0% w/v. Unit dose compositions of the invention will be sterile, but typically unpreserved. Such compositions, therefore, generally will not contain preservatives. The ophthalmic compositions of the invention may also be provided preservative free and packaged in unit dose form.

The concentration of gabapentin, a salt thereof, or an ester thereof in a pharmaceutical composition of the present invention can be in the range from about 0.0001 to about 100 mg/ml (or, alternatively, from about from about 0.001 to about 50 mg/ml, or from about 0.001 to about 30 mg/ml, or from about 0.001 to about 25 mg/ml, or from about 0.001 to about 10 mg/ml, or from about 0.001 to about 5 mg/ml, or from about 0.01 to about 30 mg/ml, or from about 0.01 to about 25 mg/ml, or from about 0.01 to about 10 mg/ml, or from about 0.1 to about 10 mg/ml, or from about 0.1 to about 5 mg/ml).

In addition, a composition of the invention can include additives such as buffers, diluents, carriers, adjuvants, or other excipients. Any pharmacologically acceptable buffer suitable for application to the eye may be used. Other agents may be employed in the composition for a variety of purposes. For example, buffering agents, preservatives, co-solvents, oils, humectants, emollients, stabilizers, or antioxidants may be employed. Water-soluble preservatives which may be employed include sodium bisulfite, sodium bisulfate, sodium thiosulfate, benzalkonium chloride, chlorobutanol, thimerosal, ethyl alcohol, methylparaben, polyvinyl alcohol, benzyl alcohol, and phenylethyl alcohol. These agents may be present in individual amounts of from about 0.001 to about 5% by weight (preferably, about 0.01% to about 2% by weight). Suitable water-soluble buffering agents that may be employed are sodium carbonate, sodium borate, sodium phosphate, sodium acetate, sodium bicarbonate, etc., for the desired route of administration. These agents may be present in amounts sufficient to maintain a pH of the system of between about 2 and about 9. As such, the buffering agent may be as much as about 5% on a weight to weight basis of the total composition. Electrolytes such as sodium chloride and potassium chloride may also be included in the formulation.

The compositions of the invention can further comprise a non-ionic surfactant, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are disclosed in Martindale, 34th ed., pp. 1411-1416 (Martindale, "The Complete Drug Reference," S.C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006. The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1, or from about 0.01 to about 0.5 weight percent). In addition, if desired, the pharmaceutical composition may also contain non-toxic auxiliary substances such as wetting or emulsifying agents, pH buffering agents, tonicity agents, liposome, mucoadhesive agents (i.e. hyaluronic acid), complexing agents (i.e. cyclodextrins, e.g. hydroxypropyl-beta--cyclodextrin), viscosizing agents (i.e. cellulose, polyvinylpyrrolidone).

The compositions of the invention are effective in the treatment of the ocular pain and/or inflammatory reactions in the eye, particularly ocular pain deriving from ocular and palpebral surgery such as photorefractive keratotomy, post-herpetic neuralgia, emergency department care of herpes zoster ophthalmicus (HZO), inflammatory processes, discomfort accompanying chronic diseases that produce irritation and pain arising from the anterior segment of the eye and conjunctiva.

The invention is illustrated in more detail in the following examples.

### EXAMPLE 1

| | |
|---|---|
| Gabapentin | 0.1 g |
| 8-methyl-N-vanillyl-6-noneamide | 0.3 g |
| Lidocaine | 0.8 g |
| Sodium dihydrogenphosphate | 0.2 g |
| Sodium chloride | 0.8 g |
| Benzalkonium chloride | 0.01 g |
| Purified water | q.s. to 100 mL |

### EXAMPLE 2

| | |
|---|---|
| Gabapentin | 0.1 g |
| Hyaluronic acid | 0.1 g |
| 8-methyl-N-vanillyl-6-noneamide | 0.3 g |
| Lidocaine | 0.8 g |
| Sodium dihydrogenphosphate | 0.2 g |
| Sodium chloride | 0.8 g |
| Benzalkonium chloride | 0.01 g |
| Purified water | q.s. to 100 mL |

### EXAMPLE 3

An in vivo study was carried out in order to evaluate the effects of the above reported formulations.

The effects of 2 formulations above described on ocular pain in rats were tested using a formalin- induced blink response assay. Sprague-Dawley rats were divided in 2 groups and treated topically with 20 µL of formulation described in the example 1 and example 2 in the right eye. After the appropriate pretreatment time of about 5 minutes, 5 µL of 0.1 % formalin was applied topically in the eye. Each rat was placed in a clear plastic box, and the number of blinks was counted for 1 minute immediately following the formalin challenge. The results of the blink response assay indicated that gabapentin inhibited the formalin-induced blink response in a dose-dependent fashion, achieving significant inhibition at the highest concentration tested (Table), and that this effect was potentiated by lidocaine and capsaicin and brimonidine in a dose dependent fashion (Table).

**Table. Effect of gabapentin-based formulations on formalin-induced blink response in rats**

| Formulation | Pre-treatment time (min) | Numbers of blinks/min (mean±S.D.) | % of inhibition |
|---|---|---|---|
| Control | 5 | 76±11 | |
| Example 1 | 5 | 41±10 | 46.1* |
| Example 2 | 5 | 38±14 | 50* |

| | | | |
|---|---|---|---|
| *p<0.01 vs. control Dunnett test | | | |

## Claims

1. A pharmaceutical composition for the topical ophthalmic administration comprising gapabentin, capsaicin and a local anaestethic as the active ingredients and at least one pharmaceutically acceptable carrier, adjuvant and/or diluent.

2. A composition according to any one of claim 1, in form of a liquid, gel, cream, lotion, liposomes, suspension, eye drops or eye drop device.

3. A composition according to claim 1 or 2 wherein the local anaesthetic is lidocaine.

4. A composition according to anyone of claims 1 to 3 further comprising hyaluronic acid or a salt thereof.

5. A composition of claims 1-4 for use in the treatment of ocular pain and/or ocular inflammation.

6. A composition for use according to claim 5 wherein the ocular pain and/or inflammatory reactions in the eye derives from ocular and palpebral surgery such as photorefractive keratotomy, post-herpetic neuralgia, emergency department care of herpes zoster ophthalmicus (HZO), inflammatory processes, discomfort accompanying chronic diseases that produce irritation and pain arising from the anterior segment of the eye and conjunctiva.

## Patentansprüche

1. Pharmazeutische Zusammensetzung zur topischen ophthalmischen Verabreichung, umfassend Gapabentin, Capsaicin und ein Lokalanästhetikum als aktive Bestandteile und mindestens einen pharmazeutisch verträglichen Träger, Hilfsstoff und/oder Verdünner.

2. Zusammensetzung nach Anspruch 1 in Form von Flüssigkeit, Gel, Creme, Lotion, Liposomen, Suspension, Augentropfen oder Augentropfvorrichtung.

3. Zusammensetzung nach Anspruch 1 oder 2, wobei das Lokalanästhetikum Lidocain ist.

4. Zusammensetzung nach einem der Ansprüche 1 bis 3 darüber hinaus umfassend Hyaluronsäure oder ein Salz davon.

5. Zusammensetzung nach den Ansprüchen 1 bis 4 zur Verwendung bei der Behandlung von Augenschmerzen und/oder Augenentzündung.

6. Zusammensetzung zur Verwendung nach Anspruch 5, wobei die Augenschmerzen und/oder entzündlichen Reaktionen im Auge entstehen durch okulare und palpebrale Operationen wie photorefraktive Keratotomie, post-herpetische Neuralgie, Notaufnahmeversorgung von Herpes Zoster ophthalmicus (HZO), entzündliche Prozesse, Beschwerden im Zusammenhang mit chronischen Erkrankungen, die Irritationen hervorrufen, und Schmerzen, die vom vorderen Segment des Auges und der Bindehaut ausgehen.

## Revendications

1. Composition pharmaceutique pour l'administration ophtalmique topique comprenant de la gapabentine, de la capsaïcine et un anesthésique local comme ingrédients actifs et au moins un véhicule, adjuvant et/ou diluant pharmaceutiquement acceptable.

2. Composition selon l'une quelconque de la revendication 1, sous forme d'un liquide, d'un gel, d'une crème, d'une lotion, de liposomes, d'une suspension, de gouttes oculaires ou d'un dispositif de pour goutte oculaire.

3. Composition selon la revendication 1 ou 2, dans laquelle l'anesthésique local est la lidocaine.

4. Composition selon l'une quelconque des revendications 1 à 3, comprenant en outre de l'acide hyaluronique ou un sel de celui-ci.

5. Composition selon les revendications 1 à 4 pour une utilisation dans le traitement d'une douleur oculaire et/ou d'une inflammation oculaire.

6. Composition pour une utilisation selon la revendication 5, dans laquelle la douleur oculaire et/ou les réactions inflammatoires dans l'oeil découlent de chirurgie oculaire et palpébrale comme une photokératectomie réfractive, d'une névralgie post-herpétique, d'un soin aux urgences d'un zona ophtalmique (HZO), de processus inflammatoires, d'un inconfort accompagnant les maladies chroniques qui produisent une irritation et une douleur provenant du segment antérieur de l'oeil et de la conjonctive.
